# EUROPEAN PATENT SPECIFICATION

(11) **EP 3 706 629 B1**
(45) Date of publication and mention of the grant of the patent: **22.04.2026**
(21) Application number: 18795525.7
(22) Date of filing: 29.10.2018
(51) Int. Cl.: A61B 5/06, A61B 5/00, A61B 5/0215, A61B 34/20

(54) **ACTIVELY TRACKED PULMONARY ARTERY CATHETERIZATION**
AKTIV VERFOLGTE PULMONALARTERIEKATHETERISIERUNG
CATHÉTÉRISATION ARTÉRIELLE PULMONAIRE ACTIVEMENT SUIVIE

(30) Priority: 09.11.2017 EP 17200765
(43) Date of publication of application: 16.09.2020
(73) Proprietor: Koninklijke Philips N.V., 5656 AG Eindhoven (NL)
(72) Inventor: WEISS, Steffen, 5656 AE Eindhoven (NL)
(74) Representative: Philips Intellectual Property & Standards
(86) International application number: PCT/EP2018/079534
(87) International publication number: WO 2019/091808

(56) References cited:
- WO-A1-2009/148317
- US-A- 5 788 647
- US-A1- 2007 055 142
- US-A1- 2014 282 008

## Description

### FIELD OF THE INVENTION

The present invention relates to the field of pulmonary artery catheterization and in particular to a pulmonary artery catheterization assistance apparatus, a pulmonary artery catheterization system, a corresponding pulmonary artery catheterization assistance method, and computer program. While the invention is particularly beneficial for right heart catheterizations, its application to further procedures is not limited.

### BACKGROUND OF THE INVENTION

Right heart catheterizations with pulmonary artery catheters (PAC) that can measure the local intravascular blood pressure and blood oxygenation are routinely performed under fluoroscopic guidance, frequently in patients or subjects with congenital heart disease and/or pulmonary hypertension.

In order to accurately evaluate, for instance, the measured blood pressure, a knowledge of the position of a tip portion of the PAC within the subject is required, i.e. the location the blood pressure is acquired has to be correlated to an anatomical location. Known approaches for effectively evaluating the blood pressure, i.e. hemodynamic data, suffer from several drawbacks, for instance the position being difficult to determine accurately, requiring manual effort of a physician and/or implying an undesired dose of ionizing radiation.

US 2007/0055142 A1 describes methods and apparatuses for guiding the positioning of a device with a position tracking sensor and pre-recorded images. Further prior art is provided by US 2014/282008 A1 and WO 2009/148317 A1.

### SUMMARY OF THE INVENTION

It has therefore been an object of the present invention to provide a pulmonary artery catheterization assistance apparatus, a pulmonary artery catheterization system. and computer program which allow for an improved evaluation of hemodynamic data in pulmonary artery catheterization. The invention is defined by the independent claims. The dependent claims define advantageous embodiments.

### BRIEF DESCRIPTION OF THE DRAWINGS

Fig. 1 shows schematically and exemplarily a pulmonary artery catheter (PAC),
Fig. 2 shows schematically and exemplarily a pulmonary artery catheterization assistance system,
Fig. 3 shows schematically and exemplarily illustrates a hemodynamic curve at different positions of the PAC,
Fig. 4 shows schematically and exemplarily a display of a segmented model of the heart, and
Fig. 5 shows schematically and exemplarily a flowchart of a pulmonary artery catheterization assistance method.

### DETAILED DESCRIPTION OF EMBODIMENTS

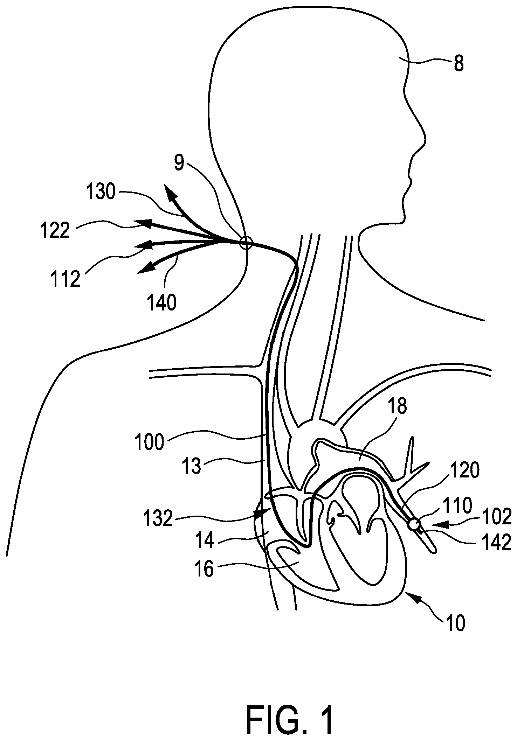

Fig. 1 schematically and exemplarily illustrates a pulmonary artery catheter 100 used in catheterization of a heart 10 of a subject 8. The catheter 100 is inserted into the body of the subject 8 at an exemplary location 9 and advanced through the subject's vascular system passing the superior vena cava 12, right atrium 14, right ventricle 16 until reaching eventually the pulmonary artery 18. Right heart catheterizations with pulmonary artery catheters, such as catheter 100, that can measure the local intravascular blood pressure and blood oxygenation, provide clinically important information on cardiac performance and systemic oxygen transport. Pulmonary artery catheters known in the art are also referred to as Swan-Ganz catheters.

Throughout the right heart catheterization, it is of utmost importance to accurately know the location of the pulmonary artery catheter 100, in particular of a distal tip 102 thereof. For this purpose, classically right heart catheterizations are performed under fluoroscopic guidance, wherein a contrast fluid is for instance received within a balloon 110 located near the distal tip 102 of the catheter 100.

The catheter comprises multiple lumens, wherein the number is four in this example. In connection with the balloon 110 is a balloon port 112, through which an inflation status of the balloon 110 can be controlled. Two further lumens include a proximal port 130 lumen and a distal lumen port 140. The distal lumen port 140 leads into a lumen connecting to a distal lumen opening 142 and the proximal lumen port 130 leads to a proximal lumen opening 132, which is arranged at a certain distance from the distal tip 102.

The distal lumen opening 142 connected to the distal lumen port 140 via a lumen allows for a measurement of the pressure present at the distal tip 102 of the catheter 100. Preferentially, a pressure sensor is connected to the lumen at the distal lumen port 140 thereof as known from conventional pressure catheters. Optionally, also at the proximal lumen port 130 a pressure sensor can be arranged.

As will be detailed in the following, in magnetic resonance (MR) guided catheterization, pressure at the proximal lumen opening 132 is not necessary to be determined. Accordingly, also the provision of the proximal lumen including proximal lumen port 130 and proximal lumen opening 132 can be omitted in MR guided applications.

Since the classically contrast-agent filled balloon requires X-ray fluoroscopy to monitor the position of the distal tip 102, ionizing radiation is required, which is a general concern to avoid or minimize if possible. In an alternative approach, which is followed according to the present invention, real-time MR guidance is used.

However, in contrast to a projection technique, MR guidance must be performed with slices of finite thickness, which causes the problem that the balloon 110 becomes invisible as soon as it leaves the current imaging slice.

For this reason, the pulmonary artery catheter 100 according to this example comprises an active tracking component 120, which comprises at least one micro-receive coil in the region of the distal tip 102 and is connected to a separate MR receiver by a MR conditional RF wire 122. The RF wire 122 is provided, for instance, in one of the lumens of the pulmonary artery catheter 100.

The active tracking component 120 is thus capable of determining the location of the pulmonary artery catheter 100 relative to an externally applied gradient field using magnetic resonances of surrounding matter or tissue. Real-time position monitoring of invasive devices using magnetic resonance is described, for instance, in the article by C. L. Doumlin et al. "Real-time position monitoring of invasive devices using magnetic resonance", Magnetic Resonance in Medicine, 1993, pages 411 to 415.

It is known that wires generally generate heat under the influence of magnetic resonance imaging, in particular during radiofrequency transmission. This can lead to damage to surrounding tissue, which is to be avoided. A wire, which is save to use in an MR system, is described, for instance, in the article by S. Weiss et al. "Transmission Line for Improved RF Safety of Interventional Devices", Magnetic Resonance in Medicine, 2005, pages 182 to 189.

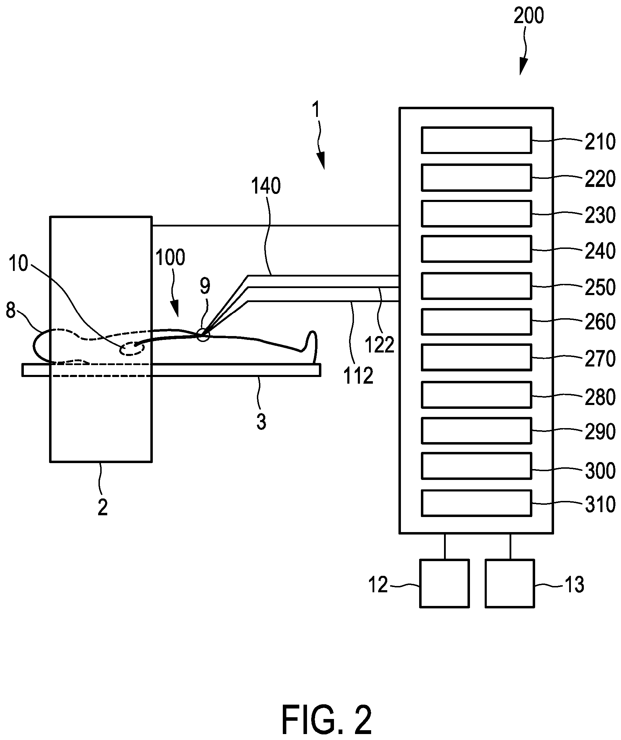

The position of the pulmonary artery catheter 100 can thus be known accurately using the MR response signals of the active tracking component 120. This information can be used advantageously to assist a user, such as a physician, in performing the catheterization. This will be explained in more detail with respect to the pulmonary artery catheterization assistance system exemplified in Fig. 2.

Right heart catheterizations are frequently performed in patients with congenital heart disease and/or pulmonary hypertension. By determining pulmonary artery pressure and flow, the pulmonary vascular resistance (PVR), which is used together with the response of PVR to nitric oxide (NO) and oxygen to assess suitability for surgery and need for vasodilator therapy, as further detailed, for instance, in the article by V. Muthurangu et al. "Novel Method of Quantifying Pulmonary Vascular Resistance by Use of Simultaneous Invasive Pressure Monitoring and Phase-Contrast Magnetic Resonance Flow", Circulation 2004, pages 826 to 834.

Conventional pulmonary artery catheters rely on the principle of thermodilution or the Fick principle to determine flow. In contrast, since the pulmonary artery catheter 100 according to the invention is magnetic resonance conditional, and since further a MR system is used for supplying the necessary excitation for the active tracking component 120, quantitative MR measurements can be used instead for accurately and directly measuring a time dependent flow through vessels, in particular the pulmonary artery. Thus, problems related to thermodilution can be avoided using the pulmonary artery catheter 100 according to the present invention. This is described in more detail, for instance, in the article by W. G. Hundley et al. "Quantitation of cardiac output with velocity-encoded, phase-difference magnetic resonance imaging", The American Journal of Cardiology, 1995, pages 1250 to 1255.

Further information helpful in right heart catheterization can be assessed accurately by fast MR imaging (MRI) with frame rates in the order of 10 Hz as described in the article by W. R. T. Witschey et al. "A Real-Time Magnetic Resonance Imaging Technique for Determining Left Ventricle Pressure-Volume Loops", Ann. Thorac Surg., 2014, pages 1597 to 1603. The time resolved simultaneous measurement of chamber volume and invasive pressure allows, among others, to acquire pressure volume loops (PVL), which are an important diagnostic tool, characterizing cardiac function and pathologies. Further relevant parameters can be derived from volume data including calculation of end-diagnostic volume (EDF), end-systolic volume (ESV), ejection fraction (EF), stroke volume (SV) and cardiac output (CO).

Pulmonary artery catheter 100 is not necessarily actively steered, but comprises balloon 110 at the distal tip 102 thereof, which is driven forward by the blood stream as known in the art. After insertion at an entry point, e.g. location 9, into a peripheral vein, the catheter is washed, as described, through the superior vena cava 12, the right atrium 14 and the ventricle 16 into the pulmonary artery 18. Further advancement is stopped due to the reduced diameter in one of the pulmonary arteries. However, catheters driven by the blood stream can make it difficult to be advanced in a controlled way to various locations within the right heart due to malformations of the heart including shunts, vascular narrowing or valve deformations, particularly in patients with congenital malfunctions of the heart. For this reason, in a further example of the pulmonary artery catheter 100, an MR-conditional active tip deflect mechanism is included in the same way as has been previously described, cf. the article by S. Weiss et al. "MR-guided cardiac radiofrequency ablation with catheter-tracked local MR lesion monitoring", Journal of Cardiovascular Magnetic Resonance, 2013, pages 1 and 2, for a different type of MR conditional catheters, namely an MR conditional RF ablation catheter.

The pulmonary artery catheter 100 will now further be described in connection with a pulmonary artery catheterization system 1 as illustrated in Fig. 2.

Fig. 2 schematically and exemplarily illustrates pulmonary artery catheterization system 1 used for carrying out and assisting a pulmonary artery catheterization of subject 8. Subject 8 is positioned on a support means 3 like a patient table within a magnetic resonance imaging apparatus 2 configured to provide MR data of the heart 10 of the subject 8. Further, MR imaging apparatus 2 is capable of providing a gradient field within the imaging region, so that active tracking component 120 of PAC 100 is capable of providing a location information of PAC 100.

In this example, the data of magnetic resonance imaging apparatus 2 and the PAC 100, more particular RF wire 122 thereof, are provided to a PAC assistance apparatus 200 according to an aspect of the invention, which will be described the following:
PAC assistance apparatus 200 comprises a location data providing unit 210, a segmented model providing unit 220, a segment determination unit 230, a hemodynamic data providing unit 240, an evaluation unit 250, an inflation status determination unit 260, a residence time evaluation unit 270, a display unit 280, a chamber volume determination unit 290, a pressure volume loop determination unit 300 and an MR data receiving unit 310. PAC assistance apparatus 200, which can for instance be implemented or integrated within a processing unit or a computer system of the MR apparatus 2, can further interact with an input unit 12 like a keyboard, a computer mouse, a touchpad, etc., and an output unit 13 like a display. It should be noted that further components of the MR imaging apparatus 2 are not illustrated in this schematic drawing, while these further components, such as a MR apparatus controller, are of course present.

Location data providing unit 210 is configured to provide location data of PAC 100, wherein in this example location data providing unit 210 is configured to receive the MR response of active tracking component 120 transmitted by RF wire 122. Location data providing unit 210 can thus be a separate MR receiver or connected to a dedicated MR receiver of the MR imaging system. The location data provided by RF wire 122 to location data providing unit 210 is indicative of a position of the actively tracked PAC 100, for instance can be translated into a location in coordinates of the MR imaging system.

Segmented model providing unit 220 provides a segmented model of at least some parts of the heart 10 of the subject 8 wherein each segment of the segmented model corresponds to a predefined region or chamber of the heart 10. More precisely, in this example the segmented model comprises a segment of the right atrium, the right ventricle and the pulmonary artery. In other examples, the segmented model can also comprise alternative or additional segments of the heart 10. Segmented model providing unit 220 provides the segmented model based on a generic model of a heart 10, which is adapted to the specific heart of the subject 8 using an MR image of MR imaging apparatus 2 prior to the catheterization procedure. This adaption of the segmented model to the individual heart of the subject 8 is preferentially done at least partly automated. Further, during catheterization, the location of the heart within the imaging space of the MR imaging apparatus 2 is preferentially correlated with the segmented model, so that the location of the tracked PAC 100 is provided in the same coordinate space as the segmented model, such that a position of the PAC 100 can be identified within the subject 8 relative to the segments of the provided segmented model.

To this end, segment determination unit 230 is configured to provide one of the segments of the segmented model as a current segment, in which the PAC 100 currently resides. More precisely, the current segment is the segment of the plurality of segments, in which the distal tip 102 of PAC 100 resides. In this example, segment determination unit 230 is configured to calculate a mean value or a centre of mass of each of the segments and determine the current segment as the segment out of the plurality of segments, which has the lowest distance to the determined position of the PAC 100.

Hemodynamic data providing unit 240 is configured to provide hemodynamic data corresponding to the location of the PAC 100. In this example, hemodynamic data providing unit 240 is configured to receive hemodynamic data from the distal tip 102 of PAC 100 using pressure information, which is measurable at a distal opening 142 thereof. The pressure information is transmitted via a distal port of the distal lumen 140, wherein a pressure sensor for digitizing and converting the hemodynamic data can be integrated into hemodynamic data providing unit 240 or be part of the pulmonary artery catheter 100. All other implementations known from pressure sensing PACs can of course be integrated in the alternative to the example shown here.

Inflation status determination unit 260 is configured to determine an inflation status of balloon 110 of PAC 100. To this end, for instance a pump connected to balloon port 112 corresponding to the port of PAC 100 communicating with the balloon 110 can be attached, which provides information indicative of the inflation status to inflation status determination unit 260. The inflation status determined by inflation status determination unit 260 comprises in this example the volume of liquid that has been injected into the balloon port 112 and preferentially comprises a derived suspected balloon status in percent of full balloon inflation. The inflation status determined by inflation status determination unit 260 can advantageously be used by the evaluation unit 250 for evaluating the hemodynamic data as will be described below.

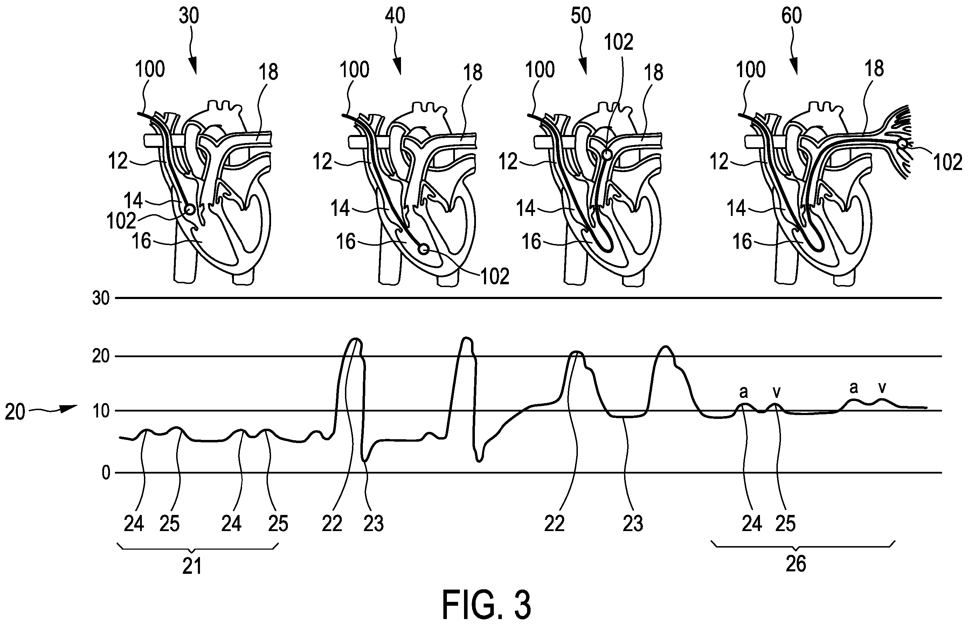

Evaluation unit 250 is configured to evaluate the hemodynamic data provided by hemodynamic data providing unit 240 in response to the segment determined by segment determination unit 230. Accordingly, evaluation unit 250 possesses additional information in addition to the hemodynamic data, namely a precise location corresponding to a segment of the heart, in which PAC 100 resides. This is based on the inventive finding that pressure time curves have typical shapes depending on in which chamber the catheter resides, i.e. depending on which segment PAC 100 is located in. Specific features in those hemodynamic data curves must thus be evaluated accordingly by evaluation unit 250. Typical hemodynamic pressure curves corresponding to specific segments or chambers of the heart 10 will be described schematically and exemplarily with reference to Fig. 3.

Fig. 3 schematically and exemplarily illustrates different positions 30, 40, 50 and 60 of distal tip 102 of PAC 100 within the heart 10 and a schematic pressure curve obtained at the respective positions in a lower diagram 20. Diagram 20 indicates a pressure on the vertical axis and both a timely and spatially evolution of the pressure sensed by distal tip 102 on a horizontal axis. The evolution on the horizontal axis would occur in case the catheter could transit from one of the respective positions 30, 40, 50 and 60 to a respective other of the positions without intermediate positions. In reality, the diagram 20 indicating a pressure curve over time would thus comprise a plurality of intermediate values.

First, with respect to position 30, distal tip 102 is located within the right atrium 14. Accordingly, segment determination unit 230 would identify the right atrium to be the current segment. In the corresponding portion of diagram 20, it can be seen that no significant pressure variations are present in the right atrium 14. Preferentially, evaluation unit 250 then, in case segment determination unit 230 determines the segment to be the right atrium, averages a mean pressure 21 over the time the catheter resides within the right atrium. Averaging in this example means the evaluation of average and standard deviation over all those R to R-peak (RR) intervals in which the catheter is present in the respective segment. In other examples, only some specific intervals of the persistence time are selected for generating the averages, as contemplated by the skilled person. Further, a- and v-waves can also be detected and averaged for the period, in which the PAC 100 resides in the right atrium 14, i.e. position 30.

Next, at position 40, catheter distal tip 102 has advanced into the right ventricle 16. In the right ventricle 16, high variations of pressure in one RR interval are present. Thus, in case evaluation unit 250 determines the current segment to be the right ventricle 16, a systolic pressure 22 and a diastolic pressure 23 can be detected and preferentially averaged. In the right ventricle, a generally expected systolic pressure would amount to 20 to 30 mm of mercury and a typically diastolic pressure would amount to 0 to 8 mm of mercury. Deviations from these values usually expected could be an indication for a pathological condition.

Next, in position 50, catheter distal tip 102 has advanced into the pulmonary artery 18. In this region, evaluation unit 250 can also be configured to automatically average and detect systolic 22 and diastolic 23 pressure values. In the pulmonary artery, different from the right ventricle, systolic values are expected to be in about the same magnitude, i.e. 20 to 30 mm of mercury, while diastolic pressures are expected to be higher, i.e. between 8 and 15 mm of mercury.

Next, in position 60, distal tip 102 is still located in a branch of the pulmonary artery 18, wherein in location 60 now balloon 110 of PAC 100 wedges the artery branch it recites in, so that a pulmonary wedge pressure can be acquired. The pulmonary artery wedge pressure is expected to be in a region of between 8 and 12 mm of mercury, i.e. higher than the right arterial pressure expected to be between 0 and 8 mm of mercury, but is devoid of variances such as systolic and diastolic variations. In contrast, only an a- and v-wave, indicated with 24 and 25, can be detected and averaged over the period, the PAC 100 resides with an inflated balloon 110. In addition, a mean wedge pressure 26 can be averaged over this period.

Returning to Fig. 2, PAC assistance of apparatus 200 further comprises the residence time evaluation unit 270 as mentioned above. Residence time evaluation unit 270 is configured to evaluate the time PAC 100 resides within a current segment. This can, for example, be done by monitoring the current segment and starting or stopping the residence time determination upon segment determination unit 230 determining a changed current segment. The residence time evaluation unit 270 then determines whether a hemodynamic data evaluated for a particular segment is enough, e.g. a minimum amount of time and/or heartbeats is evaluated or the standard deviation of the averaged measure drops below a certain threshold, and can provide an indication to the operator that the PAC 100 may be moved into the next position, i.e. the next segment, in case enough data has been collected. To this end, residence time evaluation unit 270 can be arranged to communicate with output means 13.

Chamber volume determination unit 290 is configured to determine a volume of a chamber of the heart, in particular of the right ventricle. To this end, in one example, chamber volume determination unit 290 is configured to receive MR images of at least the right ventricle of the heart and to calculate the volume of the chamber based on the received MR images.

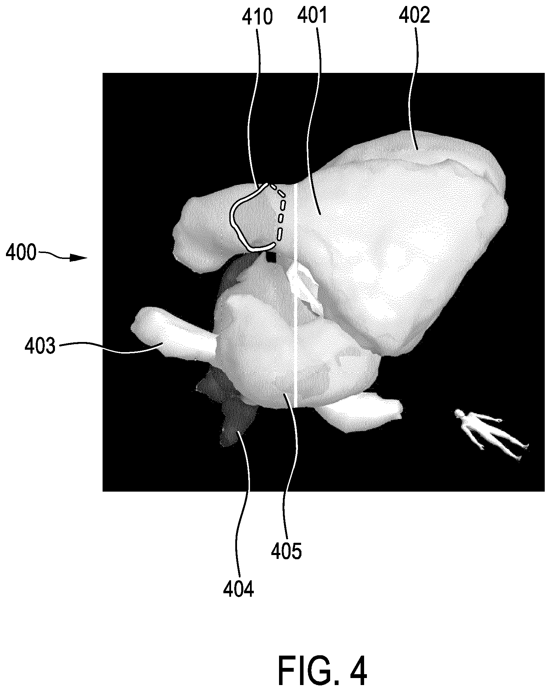

Display unit 280 is configured to generate a display for assisting a user, e.g. the operator of PAC 100, which then can be displayed on, for instance, output means 13. The generated display comprises a representation of the segmented model, wherein the segments are displayed with a respective different representation in response to the evaluation of evaluation unit 250. An example of differently represented segments will be described with reference to Fig. 4.

Fig. 4 schematically and exemplarily illustrates a display 400 of various segments 401, 402, 403, 404 and 405 of the segmented model provided by segmented model providing unit 220. The respective segments 401 to 405 are displayed with a respective different representation, wherein in this example the representation is changed to a specific representation accounting for differences in the hemodynamic data. For instance, healthy evaluation results can be represented in a different representation as unhealthy or pathologic segments. Further, segments that still lack measurements of hemodynamic data can have a different representation than those, of which already sufficient data has been evaluated. Further, mean or mean systolic, or mean diastolic pressures may be indicated per color. These are of course only examples, wherein other representations are of course contemplated.

Fig. 4 further indicates a cross section 410, in this example the pulmonary valve cross section, as an example of a cross section, for which a flow measurement can be carried out based on MR data. The use of cross section 410 for the present invention will be described with reference to an alternative of the functioning of chamber volume determination unit 290. As an alternative to the direct determination of the volume based on the MR image, chamber volume determination unit 290 can be configured to acquire a time resolved quantitative flow measurement both at the tricuspid valve cross section (not indicated in Fig. 4) and the pulmonary valve cross section 410.

Flow measurements of these cross sections are indicative of inflow and outflow of the right ventricle and the sum of time integrals of those flows is identical to the volume changes of the right ventricle overtime. In one example, segmented model providing unit 210 can be configured to further provide the locations of the required planes, e.g. the tricuspid valve and the pulmonary valve, to facilitate determination. It should be noted that this approach is only valid if there are no shunts or other obstructions present in the right ventricle.

Based on the determined chamber volume, pressure volume loop determination unit 300 is configured to measure a pressure volume loop in the right ventricle in a full scale examination. Preferentially, the determination of the pressure volume loop is triggered by the segment determination unit 230 determining the current segment to be the right ventricle. Expressed differently, the catheter 100 enters the right ventricle and then the pressure volume loop determination is started. The pressure volume loop acquisition comprises a simultaneous acquisition of pressure data, i.e. hemodynamic data, and volume data of the right ventricle obtained by the chamber volume determination unit 290.

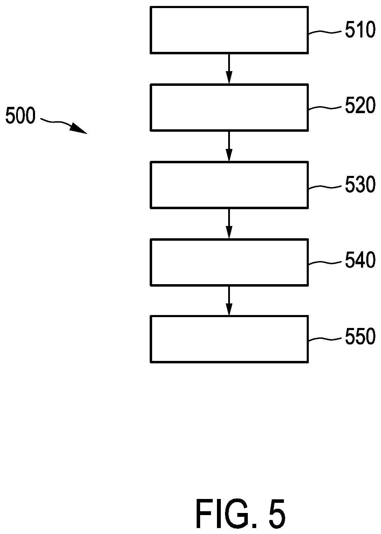

Finally, with reference to Fig. 5, a pulmonary artery catheterization assistance method 500 is schematically and exemplarily illustrated.

In first a step 510, location data of an actively tracked PAC 100 is provided. The location data is indicative of a position of the PAC.

In a step 520, a segmented model of the heart 10 of the subject 8 is provided, for instance by segmented model providing unit 220. Each segment corresponds to a predefined region, e.g. a chamber, of the heart 10.

In a step 530, a current segment of the plurality of segments is determined using the location data and the segmented model. The current segment corresponds to the location of the actively tracked PAC 100.

In a step 540, hemodynamic data corresponding to the location of the PAC 100 is provided, for instance, by hemodynamic data providing unit 240.

Finally, in a step 550, the hemodynamic data provided by hemodynamic data providing unit 240 is evaluated in reaction to the segment determined by, for instance, segment determination unit 230.

Other variations to the disclosed embodiments can be understood and effectuated by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or devices may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A pulmonary artery catheterization assistance apparatus for assisting catheterization of a heart (10) of a subject (8), wherein the pulmonary artery catheterization assistance apparatus (200) comprises:
- a location data providing unit (210) for providing location data of an actively tracked pulmonary artery catheter (100), wherein the location data is indicative of a position of the actively tracked pulmonary artery catheter (100),
- a segmented model providing unit (220) for providing a segmented model of the heart (10) of the subject (8) comprising a plurality of segments (401, 402, 403, 404, 405), wherein each segment corresponds to a predefined region of the heart (10),
- a segment determination unit (230) for determining a current segment of the plurality of segments (401, 402, 403, 404, 405) of the segmented model using the location data, wherein the current segment corresponds to the location of the actively tracked pulmonary artery catheter (100),
- a hemodynamic data providing unit (240) for providing hemodynamic data, wherein the hemodynamic data comprises blood pressure curves corresponding to the location of the actively tracked pulmonary artery catheter (100),
- an evaluation unit (250) for evaluating the blood pressure curves in reaction to the determined segment in which the actively tracked pulmonary artery catheter (100) currently resides, wherein the evaluation is segment specific to consider local differences of the blood pressure curves among the segments, such that an improved evaluation can be performed, and
- a display unit for generating a display for assisting a user, the generated display comprising a representation of the segmented model, wherein the segments are displayed with a respective different representation in response to the evaluated blood pressure curves,
wherein the segmented model providing unit (220) is configured to provide at least segments corresponding to the right atrium (14), the right ventricle (16) and the pulmonary arteries (18), and
wherein the evaluation unit (250) is configured to:
- detect and average a mean pressure from the blood pressure curves obtained in the right atrium (14) in case the determined segment corresponds to the right atrium (14),
- detect and average an a and v wave height from the blood pressure curves obtained in the right atrium (14) in case the determined segment corresponds to the right atrium (14), and
- detect and average systolic and diastolic pressure from the blood pressure curves obtained in the right ventricle (16) or the pulmonary artery (18) in case the determined segment corresponds to the right ventricle (16) or the pulmonary artery (18), respectively.

2. The pulmonary artery catheterization assistance apparatus as defined in claim 1, wherein the pulmonary artery catheterization assistance apparatus (200) further comprises
- a chamber volume determination unit (290) for determining a volume of a chamber of the heart based on provided MR image data, and
- a pressure-volume loop determination unit (300) for determining a pressure-volume loop by acquiring the volume of the chamber of the heart, determined from the chamber volume determination unit, and the hemodynamic data, provided by the hemodynamic data providing unit (240), at simultaneous times.

3. The pulmonary artery catheterization assistance apparatus as defined in claim 2, further comprising an MR imaging apparatus configured for providing the MR image data.

4. The pulmonary artery catheterization assistance apparatus as defined in claim 2 or 3, wherein the pressure-volume loop determination unit (300) is configured to initiate a pressure-volume loop acquisition in response to a change in the determined segment.

5. The pulmonary artery catheterization assistance apparatus as defined in claim 1, wherein the segment determination unit (230) is configured to determine a mean position of each segment of the segmented model and to determine the current segment as the segment with the corresponding mean position having the lowest distance to the location of the actively tracked pulmonary artery catheter (100).

6. The pulmonary artery catheterization assistance apparatus as defined in claim 1, wherein the hemodynamic data providing unit (240) is configured to provide an average of the hemodynamic data over the residence time of the actively tracked pulmonary artery catheter (100) in a segment.

7. The pulmonary artery catheterization assistance apparatus as defined in claim 1, wherein the pulmonary artery catheterization assistance apparatus (200) further comprises an inflation status determination unit (260) for determining an inflation status of the actively tracked pulmonary artery catheter (100). wherein the inflation status determination unit (260) is preferentially configured to determine the inflation status in reaction to a volume of liquid injected into a balloon port (112) of the actively tracked pulmonary artery catheter (100), and to derive the inflation status as a percentage of full balloon (110) inflation based on the volume of liquid injected.

8. The pulmonary artery catheterization assistance apparatus as defined in claim 7, wherein the inflation status determination unit (260) is configured to determine the balloon (110) to be inflated in case the inflation status exceeds a user defined threshold or maintains at a certain inflation status over a user defined minimum time.

9. The pulmonary artery catheterization assistance apparatus as defined in claim 7, wherein in case the inflation status determination unit (260) determines the balloon (110) to be inflated, the evaluation unit (250) is configured to at least one of:
- average a mean wedge pressure, and
- detect and average an a and v wave height.

10. A pulmonary artery catheterization system (1) comprising:
- a pulmonary artery catheterization assistance apparatus (200) according to any of the preceding claims,
- a magnetic resonance imaging apparatus (2) for use with the pulmonary artery catheterization assistance apparatus (200), comprising a separate magnetic resonance receiver configured to be connected to an RF wire of an actively tracked pulmonary artery catheter, and
- a pulmonary artery catheter (100) comprising
- an inflatable balloon (110) arranged at a distal portion (102) of the catheter (100),
- a distal lumen including a distal lumen opening (142) arranged at a distal portion (102) of the catheter for providing hemodynamic data corresponding to the location of the distal lumen opening (142), and
- an active tracking component (120) for providing position data indicative of a position of the catheter (100) within the magnetic resonance imaging apparatus (2), wherein the active tracking component (120) preferentially comprises at least one µ-coil and a magnetic resonance conditional wire along the catheter (100) being attached to the µ-coil.

11. A computer program for assisting catheterization of a heart (10) of a subject (8), the computer program comprising program code means for causing a pulmonary artery catheterization system (1) as defined in claim 10 to carry out the following method steps, when the computer program is run on a processing unit of the pulmonary artery catheterization system (1):
- providing (510) location data of an actively tracked pulmonary artery catheter (100), wherein the location data is indicative of a position of the actively tracked pulmonary artery catheter (100),
- providing (520) a segmented model of the heart (10) of the subject (8) comprising a plurality of segments, wherein each segment corresponds to a predefined region of the heart (10),
- determining (530) a current segment of the plurality of segments of the segmented model using the location data, wherein the current segment corresponds to the location of the actively tracked pulmonary artery catheter (100),
- providing (540) hemodynamic data comprising blood pressure curves corresponding to the location of the actively tracked pulmonary artery catheter (100),
- evaluating (550) the blood pressure curves in reaction to the determined segment in which the actively tracked pulmonary artery catheter (100) currently resides, wherein the evaluation is segment specific to consider local differences of the blood pressure curves among the segments, such that an improved evaluation can be performed, and
- displaying a representation of the segmented model to a user, wherein the segments are displayed with a respective different representation in response to the evaluated blood pressure curves,
wherein the segmented model comprises segments corresponding to at least the right atrium (14), the right ventricle (16) and the pulmonary arteries (18), and
wherein the evaluation comprises:
- detecting and averaging a mean pressure from the blood pressure curves obtained in the right atrium (14) in case the determined segment corresponds to the right atrium (14),
- detecting and averaging an a and v wave height from the blood pressure curves obtained in the right atrium (14) in case the determined segment corresponds to the right atrium (14), and
- detecting and averaging systolic and diastolic pressure from the blood pressure curves obtained in the right ventricle (16) or the pulmonary artery (18) in case the determined segment corresponds to the right ventricle (16) or the pulmonary artery (18), respectively.

## Patentansprüche

1. Einrichtung zur Unterstützung der Lungenarterienkatheterisierung zum Unterstützen der Katheterisierung eines Herzens (10) eines Subjekts (8), wobei die Einrichtung zur Unterstützung der Lungenarterienkatheterisierung (200) umfasst:
- eine Ortsdaten-Bereitstellungseinheit (210) zum Bereitstellen von Ortsdaten eines aktiv verfolgten Lungenarterienkatheters (100), wobei die Ortsdaten eine Position des aktiv verfolgten Lungenarterienkatheters (100) angeben,
- eine segmentierte Modellbereitstellungseinheit (220) zum Bereitstellen eines segmentierten Modells des Herzens (10) des Subjekts (8), umfassend eine Vielzahl von Segmenten (401, 402, 403, 404, 405), wobei jedes Segment einem vordefinierten Bereich des Herzens (10) entspricht,
- eine Segmentbestimmungseinheit (230) zum Bestimmen eines aktuellen Segments der Vielzahl von Segmenten (401, 402, 403, 404, 405) des segmentierten Modells unter Verwendung der Ortsdaten, wobei das aktuelle Segment dem Ort des aktiv verfolgten Lungenarterienkatheters (100) entspricht,
- eine hämodynamische Datenbereitstellungseinheit (240) zum Bereitstellen hämodynamischer Daten, wobei die hämodynamischen Daten Blutdruckkurven umfassen, die dem Ort des aktiv verfolgten Lungenarterienkatheters (100) entsprechen,
- eine Auswertungseinheit (250) zum Auswerten der Blutdruckkurven als Reaktion auf das bestimmte Segment, in dem sich der aktiv verfolgte Lungenarterienkatheter (100) gerade befindet, wobei die Auswertung segmentspezifisch ist, um lokale Unterschiede der Blutdruckkurven unter den Segmenten zu berücksichtigen, sodass eine verbesserte Auswertung durchgeführt werden kann, und
- eine Anzeigeeinheit zum Erzeugen einer Anzeige zur Unterstützung eines Benutzers, wobei die erzeugte Anzeige eine Darstellung des segmentierten Modells umfasst, wobei die Segmente als Reaktion auf die ausgewerteten Blutdruckkurven mit einer jeweiligen unterschiedlichen Darstellung angezeigt werden,
wobei die segmentierte Modellbereitstellungseinheit (220) konfiguriert ist, um mindestens Segmente bereitzustellen, die dem rechten Vorhof (14), der rechten Herzkammer (16) und den Lungenarterien (18) entsprechen, und
wobei die Auswertungseinheit (250) konfiguriert ist, um:
- einen mittleren Druck aus den im rechten Vorhof (14) erhaltenen Blutdruckkurven in einem Fall nachzuweisen und zu mitteln, in dem das bestimmte Segment dem rechten Vorhof (14) entspricht,
- eine a- und v-Wellenhöhe aus den im rechten Vorhof (14) erhaltenen Blutdruckkurven in einem Fall nachzuweisen und zu mitteln, in dem das bestimmte Segment dem rechten Vorhof (14) entspricht, und
- einen systolischen und diastolischen Druck aus den in der rechten Herzkammer (16) oder der Lungenarterie (18) erhaltenen Blutdruckkurven in einem Fall nachzuweisen und zu mitteln, in dem das bestimmte Segment jeweils dem der rechten Herzkammer (16) oder der Lungenarterie (18) entspricht,

2. Einrichtung zur Unterstützung der Lungenarterienkatheterisierung nach Anspruch 1, wobei die Einrichtung zur Unterstützung der Lungenarterienkatheterisierung (200) weiter umfasst:
- eine Kammervolumenbestimmungseinheit (290) zum Bestimmen eines Volumens einer Kammer des Herzens basierend auf bereitgestellten MR-Bilddaten, und
- eine Druck-Volumen-Schleifenbestimmungseinheit (300) zum Bestimmen einer Druck-Volumen-Schleife durch gleichzeitiges Erfassen des Volumens der Kammer des Herzens, das von der Kammervolumenbestimmungseinheit bestimmt wird, und der hämodynamischen Daten, die von der hämodynamischen Datenbereitstellungseinheit (240) bereitgestellt werden.

3. Einrichtung zur Unterstützung der Lungenarterienkatheterisierung nach Anspruch 2, die weiter eine MR-Bildgebungseinrichtung umfasst, die zum Bereitstellen der MR-Bilddaten konfiguriert ist.

4. Einrichtung zur Unterstützung der Lungenarterienkatheterisierung nach Anspruch 2 oder 3, wobei die Druck-Volumen-Schleifen-Bestimmungseinheit (300) konfiguriert ist, um eine Druck-Volumen-Schleifen-Erfassung als Reaktion auf eine Änderung des bestimmten Segments zu initiieren.

5. Einrichtung zur Unterstützung der Lungenarterienkatheterisierung nach Anspruch 1, wobei die Segmentbestimmungseinheit (230) konfiguriert ist, um eine mittlere Position jedes Segments des segmentierten Modells zu bestimmen, und um das aktuelle Segment als das Segment mit der entsprechenden mittleren Position zu bestimmen, die den geringsten Abstand zum Ort des aktiv verfolgten Lungenarterienkatheters (100) aufweist.

6. Einrichtung zur Unterstützung der Lungenarterienkatheterisierung nach Anspruch 1, wobei die hämodynamische Datenbereitstellungseinheit (240) konfiguriert ist, um einen Mittelwert der hämodynamischen Daten über die Verweilzeit des aktiv verfolgten Lungenarterienkatheters (100) in einem Segment bereitzustellen.

7. Einrichtung zur Unterstützung der Lungenarterienkatheterisierung nach Anspruch 1, wobei die Einrichtung zur Unterstützung der Lungenarterienkatheterisierung (200) weiter eine Einheit (260) zur Bestimmung des Aufblaszustands des aktiv verfolgten Lungenarterienkatheters (100) umfasst, wobei die Einheit (260) zur Bestimmung des Aufblaszustands vorzugsweise konfiguriert ist, um den Aufblaszustand als Reaktion auf ein in einen Ballonanschluss (112) des aktiv verfolgten Lungenarterienkatheters (100) injiziertes Flüssigkeitsvolumen zu bestimmen, und um den Aufblaszustand als Prozentsatz einer vollständigen Ballonaufblasung (110) basierend auf dem injizierten Flüssigkeitsvolumen abzuleiten.

8. Einrichtung zur Unterstützung der Lungenarterienkatheterisierung nach Anspruch 7, wobei die Einheit (260) zur Bestimmung des Aufblaszustands konfiguriert ist, um in einem Fall zu bestimmen, dass der Ballon (110) aufgeblasen ist, in dem der Aufblaszustand einen vom Benutzer definierten Schwellenwert überschreitet oder über eine vom Benutzer definierte Mindestzeit auf einem bestimmten Aufblaszustand verbleibt.

9. Einrichtung zur Unterstützung der Lungenarterienkatheterisierung nach Anspruch 7, wobei in einem Fall, in dem die Einheit (260) zur Bestimmung des Aufblaszustands bestimmt, dass der Ballon (110) aufgeblasen ist, die Auswertungseinheit (250) konfiguriert ist zum mindestens eines von:
- Mitteln eines mittleren Verkeilungsdrucks, und
- Nachweisen und Mitteln einer a- und v-Wellenhöhe.

10. System zur Lungenarterienkatheterisierung (1), umfassend:
- eine Einrichtung zur Unterstützung der Lungenarterienkatheterisierung (200) nach einem der vorstehenden Ansprüche,
- eine Magnetresonanz-Bildgebungseinrichtung (2) zur Verwendung mit der Einrichtung zur Unterstützung der Lungenarterienkatheterisierung (200), umfassend einen separaten Magnetresonanzempfänger, der konfiguriert ist, um mit einem RF-Draht eines aktiv verfolgten Lungenarterienkatheters verbunden zu werden, und
- einen Lungenarterienkatheter (100), umfassend
- einen aufblasbaren Ballon (110), der an einem distalen Abschnitt (102) des Katheters (100) angeordnet ist,
- ein distales Lumen, das eine distale Lumenöffnung (142) beinhaltet, die an einem distalen Abschnitt (102) des Katheters angeordnet ist, zum Bereitstellen hämodynamischer Daten, die dem Ort der distalen Lumenöffnung (142) entsprechen, und
- eine aktive Verfolgungskomponente (120) zum Bereitstellen von Positionsdaten, die eine Position des Katheters (100) innerhalb der Magnetresonanz-Bildgebungseinrichtung (2) angeben, wobei die aktive Verfolgungskomponente (120) vorzugsweise mindestens eine µ-Spule und einen entlang des Katheters (100) verlaufenden, magnetresonanzbedingten Draht umfasst, der an der µ-Spule befestigt ist.

11. Computerprogramm zum Unterstützen einer Katheterisierung eines Herzens (10) eines Subjekts (8), wobei das Computerprogramm Programmcodemittel zum Veranlassen eines Systems zur Lungenarterienkatheterisierung (1) nach Anspruch 10 umfasst, die folgenden Verfahrensschritte durchzuführen, wenn das Computerprogramm auf einer Verarbeitungseinheit des Systems zur Lungenarterienkatheterisierung (1) läuft:
- Bereitstellen (510) von Ortsdaten eines aktiv verfolgten Lungenarterienkatheters (100), wobei die Ortsdaten eine Position des aktiv verfolgten Lungenarterienkatheters (100) angeben,
- Bereitstellen (520) eines segmentierten Modells des Herzens (10) des Subjekts (8), umfassend eine Vielzahl von Segmenten, wobei jedes Segment einem vordefinierten Bereich des Herzens (10) entspricht,
- Bestimmen (530) eines aktuellen Segments der Vielzahl von Segmenten des segmentierten Modells unter Verwendung der Ortsdaten, wobei das aktuelle Segment dem Ort des aktiv verfolgten Lungenarterienkatheters (100) entspricht,
- Bereitstellen (540) hämodynamischer Daten, die Blutdruckkurven umfassen, die dem Ort des aktiv verfolgten Lungenarterienkatheters (100) entsprechen,
- Auswerten (550) der Blutdruckkurven als Reaktion auf das bestimmte Segment, in dem sich der aktiv verfolgte Lungenarterienkatheter (100) gerade befindet, wobei die Auswertung segmentspezifisch ist, um lokale Unterschiede der Blutdruckkurven unter den Segmenten zu berücksichtigen, sodass eine verbesserte Auswertung durchgeführt werden kann, und
- Anzeigen einer Darstellung des segmentierten Modells für einen Benutzer, wobei die Segmente als Reaktion auf die ausgewerteten Blutdruckkurven mit einer jeweiligen unterschiedlichen Darstellung angezeigt werden,
wobei das segmentierte Modell Segmente umfasst, die mindestens dem rechten Vorhof (14), der rechten Herzkammer (16) und den Lungenarterien (18) entsprechen, und
wobei die Auswertung umfasst:
- Nachweisen und Mitteln eines mittleren Drucks aus den im rechten Vorhof (14) erhaltenen Blutdruckkurven in einem Fall, in dem das bestimmte Segment dem rechten Vorhof (14) entspricht,
- Nachweisen und Mitteln einer a- und v-Wellenhöhe aus den im rechten Vorhof (14) erhaltenen Blutdruckkurven in einem Fall, in dem das bestimmte Segment dem rechten Vorhof (14) entspricht, und
- Nachweisen und Mitteln eines systolischen und diastolischen Drucks aus den in der rechten Herzkammer (16) oder der Lungenarterie (18) erhaltenen Blutdruckkurven in einem Fall, in dem das bestimmte Segment jeweils dem der rechten Herzkammer (16) oder der Lungenarterie (18) entspricht.

## Revendications

1. Appareil d'assistance au cathétérisme de l'artère pulmonaire destiné à faciliter le cathétérisme d'un cœur (10) d'un sujet (8), dans lequel l'appareil d'assistance au cathétérisme de l'artère pulmonaire (200) comprend :
- une unité de fourniture de données de localisation (210) destinée à fournir des données de localisation d'un cathéter d'artère pulmonaire suivi activement (100), dans lequel les données de localisation indiquent une position du cathéter d'artère pulmonaire suivi activement (100),
- une unité de fourniture de modèle segmenté (220) destinée à fournir un modèle segmenté du cœur (10) du sujet (8) comprenant une pluralité de segments (401, 402, 403, 404, 405), dans lequel chaque segment correspond à une région prédéfinie du cœur (10),
- une unité de détermination de segment (230) destinée à déterminer un segment actuel de la pluralité de segments (401, 402, 403, 404, 405) du modèle segmenté à l'aide des données de localisation, dans lequel le segment actuel correspond à l'emplacement du cathéter d'artère pulmonaire suivi activement (100),
- une unité de fourniture de données hémodynamiques (240) destinée à fournir des données hémodynamiques, dans lequel les données hémodynamiques comprennent des courbes de pression artérielle correspondant à la localisation du cathéter d'artère pulmonaire suivi activement (100),
- une unité d'évaluation (250) destinée à évaluer les courbes de pression artérielle en réaction au segment déterminé dans lequel se trouve actuellement le cathéter d'artère pulmonaire suivi activement (100), dans lequel l'évaluation est spécifique au segment pour prendre en compte des différences locales des courbes de pression artérielle entre les segments, de telle sorte qu'une évaluation améliorée peut être réalisée, et
- une unité d'affichage destinée à générer un affichage destiné à assister un utilisateur, l'affichage généré comprenant une représentation du modèle segmenté, dans lequel les segments sont affichés avec une représentation différente respective en réponse aux courbes de pression artérielle évaluées,
dans lequel l'unité de fourniture de modèle segmenté (220) est configurée pour fournir au moins des segments correspondant à l'atrium droit (14), au ventricule droit (16) et aux artères pulmonaires (18), et
dans lequel l'unité d'évaluation (250) est configurée pour :
- détecter et calculer une pression moyenne à partir des courbes de pression artérielle obtenues dans l'atrium droit (14) dans le cas où le segment déterminé correspond à l'atrium droit (14),
- détecter et calculer une moyenne de l'amplitude des ondes a et v à partir des courbes de pression artérielle obtenues dans l'atrium droit (14) dans le cas où le segment déterminé correspond à l'atrium droit (14), et
- détecter et calculer une pression systolique et diastolique moyenne à partir des courbes de pression artérielle obtenues dans le ventricule droit (16) ou l'artère pulmonaire (18) dans le cas où le segment déterminé correspond au ventricule droit (16) ou à l'artère pulmonaire (18), respectivement.

2. Appareil d'assistance au cathétérisme de l'artère pulmonaire selon la revendication 1, dans lequel l'appareil d'assistance au cathétérisme de l'artère pulmonaire (200) comprend en outre
- une unité de détermination du volume de cavité (290) destinée à déterminer un volume d'une cavité du cœur sur la base des données d'imagerie IRM fournies, et
- une unité de détermination de boucle pression-volume (300) destinée à déterminer une boucle pression-volume en acquérant simultanément le volume de la cavité du cœur, déterminé à partir de l'unité de détermination du volume de la cavité et les données hémodynamiques, fournies par l'unité de fourniture de données hémodynamiques (240), à des moments simultanés.

3. Appareil d'assistance au cathétérisme de l'artère pulmonaire selon la revendication 2, comprenant en outre un appareil d'imagerie IRM configuré pour fournir les données d'image IRM.

4. Appareil d'assistance au cathétérisme de l'artère pulmonaire selon la revendication 2 ou 3, dans lequel l'unité de détermination de la boucle pression-volume (300) est configurée pour initier une acquisition de boucle pression-volume en réponse à un changement dans le segment déterminé.

5. Appareil d'assistance au cathétérisme de l'artère pulmonaire selon la revendication 1, dans lequel l'unité de détermination de segment (230) est configurée pour déterminer une position moyenne de chaque segment du modèle segmenté et pour déterminer le segment actuel comme le segment dont la position moyenne correspondante présente la plus faible distance par rapport à la localisation du cathéter d'artère pulmonaire suivi activement (100).

6. Appareil d'assistance au cathétérisme de l'artère pulmonaire selon la revendication 1, dans lequel l'unité de fourniture de données hémodynamiques (240) est configurée pour fournir une moyenne des données hémodynamiques sur le temps de résidence du cathéter d'artère pulmonaire suivi activement (100) dans un segment.

7. Appareil d'assistance au cathétérisme de l'artère pulmonaire selon la revendication 1, dans lequel l'appareil d'assistance au cathétérisme de l'artère pulmonaire (200) comprend en outre une unité de détermination de l'état de gonflage (260) pour déterminer l'état de gonflage du cathéter d'artère pulmonaire suivi activement (100), dans lequel l'unité de détermination de l'état de gonflage 260) est de préférence configurée pour déterminer l'état de gonflage en réaction à un volume de liquide injecté dans un orifice à ballonnet (112) du cathéter d'artère pulmonaire suivi activement (100) et pour déduire l'état de gonflage en pourcentage du gonflage complet du ballonnet (110) sur la base du volume de liquide injecté.

8. Appareil d'assistance au cathétérisme de l'artère pulmonaire selon la revendication 7, dans lequel l'unité de détermination de l'état de gonflage (260) est configurée pour déterminer le ballonnet (110) à gonfler dans le cas où l'état de gonflage dépasse un seuil défini par l'utilisateur ou se maintient à un certain état de gonflage pendant une durée minimale définie par l'utilisateur.

9. Appareil d'assistance au cathétérisme de l'artère pulmonaire selon la revendication 7, dans lequel, dans le cas où l'unité de détermination de l'état de gonflage (260) détermine que le ballonnet (110) doit être gonflé, l'unité d'évaluation (250) est configurée pour au moins l'une des opérations suivantes :
- le moyennage d'une pression de coin moyenne, et
- la détection et le calcul d'une moyenne d'une hauteur des ondes a et v.

10. Système de cathétérisme de l'artère pulmonaire (1) comprenant :
- un appareil d'assistance au cathétérisme de l'artère pulmonaire (200) selon l'une quelconque des revendications précédentes,
- un appareil d'imagerie par résonance magnétique (2) destiné à être utilisé avec l'appareil d'assistance au cathétérisme de l'artère pulmonaire (200), comprenant un récepteur de résonance magnétique séparé configuré pour être connecté à un fil RF d'un cathéter d'artère pulmonaire à suivi actif, et
- un cathéter d'artère pulmonaire (100) comprenant
- un ballonnet gonflable (110) agencé au niveau d'une partie distale (102) du cathéter (100),
- une lumière distale incluant une ouverture de lumière distale (142) agencée dans une portion distale (102) du cathéter pour fournir des données hémodynamiques correspondant à l'emplacement de l'ouverture de lumière distale (142), et
- un composant de suivi actif (120) destiné à fournir des données de position indicatives d'une position du cathéter (100) dans l'appareil d'imagerie par résonance magnétique (2), dans lequel le composant de suivi actif (120) comprend de préférence au moins une µ-bobine et un fil conditionnel pour résonance magnétique le long du cathéter (100) qui est fixé à la µ-bobine.

11. Programme informatique destiné à assister le cathétérisme d'un cœur (10) d'un sujet (8), le programme informatique comprenant des moyens de code de programme destinés à amener un système de cathétérisme de l'artère pulmonaire (1) selon la revendication 10 à effectuer les étapes de procédé suivantes, lorsque le programme informatique est exécuté sur une unité de traitement du système de cathétérisme de l'artère pulmonaire (1) :
- la fourniture (510) de données de localisation d'un cathéter d'artère pulmonaire suivi activement (100), dans lequel les données de localisation indiquent une position du cathéter d'artère pulmonaire suivi activement (100),
- la fourniture (520) d'un modèle segmenté du cœur (10) du sujet (8) comprenant une pluralité de segments, dans lequel chaque segment correspond à une région prédéfinie du cœur (10),
- la détermination (530) d'un segment actuel de la pluralité de segments du modèle segmenté à l'aide des données de localisation, dans lequel le segment actuel correspond à la localisation du cathéter d'artère pulmonaire suivi activement (100),
- la fourniture (540) de données hémodynamiques comprenant des courbes de pression artérielle correspondant à la localisation du cathéter d'artère pulmonaire suivi activement (100),
- l'évaluation (550) des courbes de pression artérielle en réaction au segment déterminé dans lequel se trouve actuellement le cathéter d'artère pulmonaire suivi activement (100), dans lequel l'évaluation est spécifique au segment pour prendre en compte des différences locales des courbes de pression artérielle entre les segments, de telle sorte qu'une évaluation améliorée peut être réalisée, et
- l'affichage d'une représentation du modèle segmenté à un utilisateur, dans lequel les segments sont affichés avec une représentation différente respective en réponse aux courbes de pression artérielle évaluées,
dans lequel le modèle segmenté comprend des segments correspondant au moins à l'atrium droit (14), au ventricule droit (16) et aux artères pulmonaires (18), et
dans lequel l'évaluation comprend :
- la détection et le calcul d'une moyenne d'une pression moyenne à partir des courbes de pression sanguine obtenues dans l'atrium droit (14) dans le cas où le segment déterminé correspond à l'atrium droit (14),
- la détection et le calcul d'une moyenne d'une amplitude des ondes a et v à partir des courbes de pression artérielle obtenues dans l'atrium droit (14) dans le cas où le segment déterminé correspond à l'atrium droit (14), et
- la détection et le calcul d'une moyenne de la pression systolique et diastolique à partir des courbes de pression artérielle obtenues dans le ventricule droit (16) ou l'artère pulmonaire (18) dans le cas où le segment déterminé correspond respectivement au ventricule droit (16) ou à l'artère pulmonaire (18).
